(19) Europäisches Patentamt
European Patent Office
Office européen des brevets

(11) Publication number: **0 184 822 A2**

(12) **EUROPEAN PATENT APPLICATION**

(21) Application number: **85115720.6**

(22) Date of filing: **10.12.85**

(51) Int. Cl.⁴: **C 07 C 103/28**, C 07 C 143/80, C 07 C 103/30, C 07 D 213/56, A 61 K 31/63, A 61 K 31/165, A 61 K 31/16, C 07 D 307/54, C 07 D 333/24, A 61 K 31/34

(30) Priority: **11.12.84 JP 261197/84**

(43) Date of publication of application: **18.06.86 Bulletin 86/25**

(84) Designated Contracting States: **AT BE CH DE FR GB IT LI NL SE**

(71) Applicant: **SUMITOMO PHARMACEUTICALS COMPANY, LIMITED, 40, Dosho-machi 2-chome, Higashi-ku Osaka-shi Osaka-fu (JP)**

(72) Inventor: **Kawakami, Hajime, 4-15-205, Ryodo-cho, Nishinomiya-shi Hyogo-ken (JP)**
Inventor: **Hashimoto, Katsuhiro, 11-2, Kamisanjo-cho Hyogo-ku, Kobe-shi Hyogo-ken (JP)**
Inventor: **Tamoto, Katsumi, 10-2-246, Sonehigashi-machi 2-chome, Toyonaka-shi Osaka-fu (JP)**
Inventor: **Ono, Keiichi, 10-4, Momoyamadai 3-chome, Sakai-shi Osaka-fu (JP)**
Inventor: **Yamamoto, Michihiro, 16-40-309, Takagihigashi-machi, Nishinomiya-shi Hyogo-ken (JP)**

(74) Representative: **Vossius Vossius Tauchner Heunemann Rauh, Siebertstrasse 4 P.O. Box 86 07 67, D-8000 München 86 (DE)**

(54) **Novel anilide derivatives of substituted arylacetic acids.**

(57) A compound of the formula I:

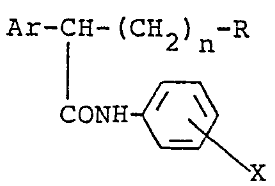

(I)

wherein Ar is a heterocyclic group or an aromatic hydrocarbon group optionally having one or two substituents selected from the group consisting of halogen, $C_1$–$C_4$ alkyl, $C_1$–$C_4$ alkoxy, nitro, cyano, di($C_1$–$C_4$) alkylamino, benzyloxy, hydroxy, $C_1$–$C_4$ alkylthio, $C_1$–$C_4$ alkylsulfinyl, $C_1$–$C_4$ alkylsulfonyl and $C_1$–$C_4$ alkanoylamino, X is a hydroxyl group or either one of the following groups:

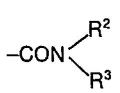 or 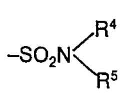

(in which $R^2$, $R^3$, $R^4$ and $R^5$ are each independently hydrogen or $C_1$–$C_4$ alkyl or, when $R^2$ and $R^3$ or $R^4$ and $R^5$ are taken together with the adjacent nitrogen atom to which they are attached, they represent pyrrolidino, piperidino, morpholino, N-($C_1$–$C_4$)-alkylpiperazino or N-phenylpiperazino), R is an aromatic hydrocarbon or heterocyclic group and n is an integer of 1 to 4, or its acid addition salt, which is useful as a cerebral metabolism stimulant or a nootropic agent.

EP 0 184 822 A2

Our Ref: U 209 EP
Sumitomo Pharmaceuticals
Company, Limited
Case: 611411

0 184 822

December 10, 1985

# NOVEL ANILIDE DERIVATIVES OF SUBSTITUTED ARYLACETIC ACIDS

The present invention relates to anilide derivatives of substituted arylacetic acids, and their production and use. More particularly, it relates to substituted arylacetic acid anilides and their acid addition salts, and their production and use.

Said substituted arylacetic acid anilides (hereinafter referred to as "anilide(s)") are represented by the formula:

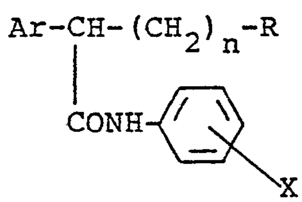

(I)

wherein Ar is a heterocyclic group or an aromatic hydrocarbon group optionally having one or two substituents selected from the group consisting of halogen, $C_1$-$C_4$ alkyl, $C_1$-$C_4$ alkoxy, nitro, cyano, di($C_1$-$C_4$)alkylamino, benzyloxy, hydroxy, $C_1$-$C_4$ alkylthio, $C_1$-$C_4$ alkylsulfinyl, $C_1$-$C_4$ alkylsulfonyl and $C_1$-$C_4$ alkanoylamino, X is a hydroxyl group or either one of the following groups: 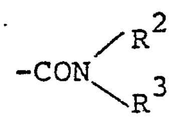 or 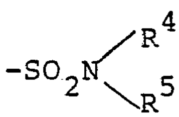 (in which $R^2$, $R^3$, $R^4$ and $R^5$ are each independently hydrogen or $C_1$-$C_4$ alkyl or, when $R^2$ and $R^3$ or $R^4$ and $R^5$ are taken together with the adjacent nitrogen atom to which they are attached, they represent pyrrolidino, piperidino, morpholino, N-($C_1$-$C_4$)alkylpiperazino or N-phenylpiperazino), R is an aromatic hydrocarbon or heterocyclic group and n is an integer of 1 to 4.

In the significances as defined above, the "aromatic hydrocarbon group" includes an aromatic carbocyclic ring having not more than 14 carbon atoms, and its typical examples are phenyl and naphthyl. The "heterocyclic group" includes a 5 or 6-membered heterocyclic ring having at least one of nitro, oxygen and sulfur as the hetero atom(s), and its specific examples are pyridyl, furyl, thienyl, etc. The term "halogen" includes fluorine, chlorine, bromine, and iodine. The term "$C_1$-$C_4$ alkyl" is intended to mean a straight or branched chain alkyl group having 1 to 4 carbon atoms (e.g. methyl, ethyl, n-propyl, isopropyl, n-butyl, sec-butyl). Likewise, the term "$C_1$-$C_4$ alkoxy" is intended to mean a straight or branched chain alkoxy group having 1 to 4 carbon atoms (e.g. methoxy, ethoxy, n-propoxy, isopropoxy, n-butoxy, sec-butoxy). The term "$C_1$-$C_4$ alkanoyl" means a straight or branched chain alkanoyl group having 1 to 4 carbon atoms (e.g. methanoyl, ethanoyl, n-propanoyl, isopropanoyl, n-butanoyl, sec-butanoyl, etc.)

Hitherto, it is known that the phenylacetanilide compound of the formula:

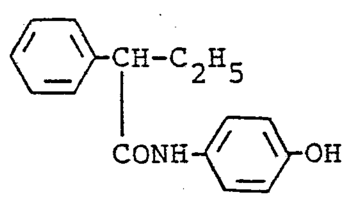

exhibits some pharmacological activities such as choleretic, hypocholesterolemic and peripheral analgesic activities [cf.

Arch.Intern.Pharmacodynamie, 116, 136-153 (1958); ibid., 116, 154-169 (1958); Farmaco (Paria) Ed. Sci., 13, 825-829 (1958)].

It has now unexpectedly been found that the anilides (I) of the invention have excellent pharmacological activities, particularly an antihypoxic activity, and are therapeutically useful for treatment of cerebral arteriosclerosis, senile mental indolence, etc. as the results of cerebral insufficiency. Thus, they are promising as nootropic agents.

From this point of view, preferred are those of the formula (I) wherein Ar and R are phenyl, X is $-CONH_2$, $-SO_2NH_2$ or hydroxyl and n is 3 or 4. Particularly preferred are those of the formula (I) wherein Ar and R are phenyl, X is $-CONH_2$ or hydoxyl and n is 3.

Accordingly, a basic object of the present invention is to provide the novel anilides (I) having an antihypoxic activity optionally with any other pharmacological activity. Another object of this invention is to provide a process for producing the anilides (I). These and other objects will be apparent to those skilled in the art to which the prevent invention pertains from the foregoing and subsequent descriptions.

According to the present invention, the anilides (I) are obtainable, for instance, by (a) reacting a substituted arylacetic acid of the formula:

$$Ar-\underset{\displaystyle COOH}{\underset{|}{CH}}-(CH_2)_n-R \qquad (II)$$

wherein Ar, R and n are each as defined above, or its reactive derivative at the -COOH group (e.g. acid halide, acid anhydride, acid ester) with a substituted aniline of the formula:

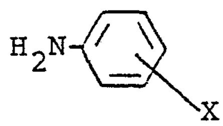

(III)

wherein X is as defined above, or (b) reacting the reactive derivative at the -COOH or $-SO_3H$ group (e.g. acid halide, acid anhydride, acid ester) of either one of substituted arylacetic acid anilides of the formulas:

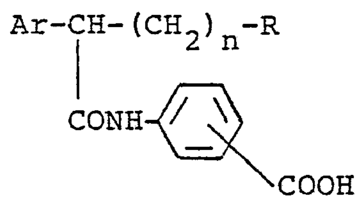

(IV)

or

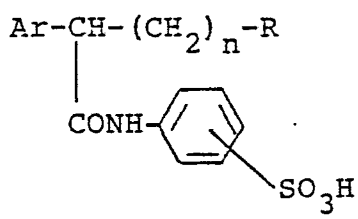

(V)

wherein Ar, R and n are each as defined above with an amine of either one of the formulas:

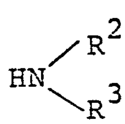

(VI)

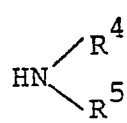

(VII)

wherein $R^2$, $R^3$, $R^4$ and $R^5$ are each as defined above.

When the reaction is carried out using the substituted arylacetic acid (II), the presence of a condensing agent (e.g. dicyclohexylcarbodiimide) in the reaction system is favorable. When the reaction is effected using the

reactive derivative of the substituted arylacetic acid (II) or the substituted arylacetic acid anilide (IV) or (V), the presence of an acid-accepting agent such as an organic or inorganic base (e.g. alkali hydroxide, alkali carbonate, triethylamine, pyridine) in the reaction system is preferable.

The reaction is normally carried out in an inert solvent, of which examples are water, alcohols (e.g. methanol, ethanol), ethers (e.g. tetrahydrofuran, dioxane), aromatic hydrocarbons (e.g. benzene, toluene), ketones (e.g. acetone, methylethylketone), etc. When the reaction is effected with the substituted arylacetic acid (II) or the substituted arylacetic acid aniline (IV) or (V) in the form of its acid halide, the use of an inert solvent chosen from amides (e.g. dimethylformamide, hexamethylphosphortriamide) is particularly favorable. Depending on the combination of the reagents, the inert solvent is not necessarily required to use.

The reaction is usually conducted at a temperature of -50°C to the refluxing temperature of the reaction system, preferably from -10 to 50°C, when an inert solvent is used.

The starting compounds (II), (IV) and (V) may be prepared as shown in the following reaction scheme:

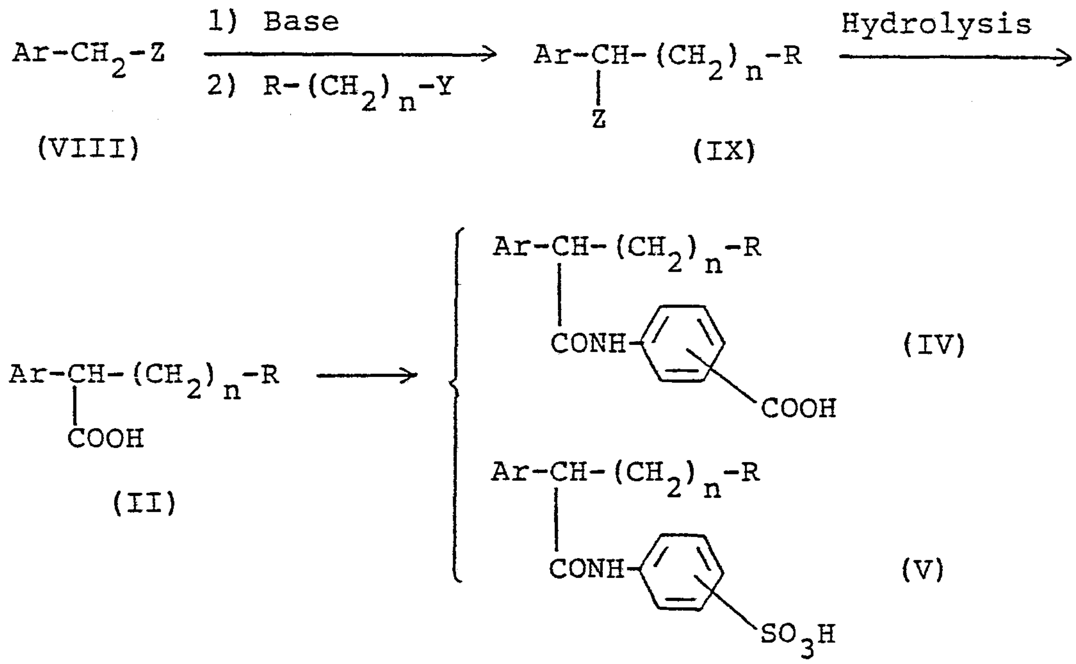


(VIII) (IX)

(II) (IV) (V)

wherein Ar, R and n are each as defined above, Z is $-COO-(C_1-C_4)$alkyl or -CN and Y is a halogen atom (e.g. chlorine, bromine).

In the foregoing scheme, the conversion of the compound (VIII) into the compound (IX) can be accomplished by alkylation of the former with an alkylating agent of the formula: $R-(CH_2)_n-Y$ in an inert solvent (e.g. benzene, toluene, dimethylformamide, dimethylsulfoxide) in the presence of a base (e.g. alkali metal alkoxide, alkali metal amide, alkali metal hydride) at a temperature ranging from 20°C to the refluxing temperature of the reaction system. The subsequent hydrolysis of the compound (IX) to the compound (II) may be carried out by a per se conventinal procedure for hydrolysis. The next condensation of the compound (II) with the aniline bearing -COOH or $-SO_3H$ to the compound (IV) or (V) may be performed in the same manner as in the condensation of the compound (II) with the substituted aniline (III) or of the compound (IV) or (V) with the amine (VI) or (VII).

The optical isomers of the anilides (I) are also included in the scope of the invention. In order to prepare such optically active compounds (I), the racemic compound (II) may be subjected to optical resolution through the step of formation of the salts with optically active amines (e.g. alpha-methylbenzylamine, cinchonine, cinchonidine, quinine, quinidine, brucine), followed by reaction of the resulting optically active compound (II) (i.e. the (+)-isomer or the (-)-isomer) with the substituted aniline (III).

The acid addition salts of the anilides (I) may be prepared in a per se conventional procedure for salt formation with acids. Examples of the acids are inorganic acids (e.g. hydrochloric acid, hydrobromic acid, sulfuric acid, phosphoric acid) and organic acids (e.g. acetic acid, oxalic acid, propionic acid, tartaric acid, citric acid, maleic acid, fumaric acid, methanesulfonic acid). The acid addition salts are preferred to be physiologically acceptable.

The anilides (I) as well as their acid addition salts can be administered orally or parenterally in the form of conventional pharmaceutical preparations. For instance, they can be administered orally in the form of conventional solid pharmaceutical preparations such as tablets, capsules, syrups and suspensions. Alternatively, they can be administered parenterally by injection in the form of conventional liquid pharmaceutical preparations such as solutions,

emulsions, suspensions, etc. Also, they may be directly applied to rectum in the form of suppository. Further, the preparations may contain physiologically acceptable carriers, excipients, activators, binding agents, stabilizers, etc. In case of injections, physiologically acceptable buffers, solubilizing agents or isotonic agents may be incorporated therein. The daily dosage may vary depending upon the symptom of disease, the age and bodyweight of patient, the administration route, etc., and the normal dosage to a human adult is between 10 and 200 mg dividing in one to several times per day.

Practical and presently preferred embodiments of the invention are illustratively shown in the following examples, which are not intended to limit the scope of the invention thereto.

Example 1

A mixture of alpha-benzylbenzeneacetic acid (2 g) and thionyl chloride (2.1 g) was heated under reflux for 1 hour, followed by distillation to remove excessive thionyl chloride. The residue was dissolved in tetrahydrofuran (10 ml) and dropwise added to a solution of 3-aminobenzamide (1.44 g) and triethylamine (2.68 g) in tetrahydrofuran (30 ml), followed by stirring for 1 hour. The reaction mixture was poured into water and extracted with ethyl acetate. The extract was washed successively with an aqueous sodium bicarbonate solution, 1N hydrochloric acid, water and brine, dried over anhydrous magnesium sulfate and concentrated. The residue was treated with methanol, collected by

filtration and dried to give alpha-benzyl-N-(3-carbamoylphenyl)benzeneacetamide. m.p., 182 - 184°C.

Examples 2 to 42

In the same manner as in Example 1, there were produced the compounds as shown in Table 1.

Table 1

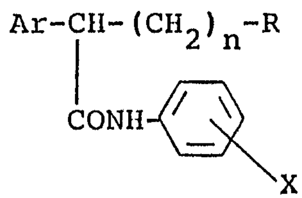

(I)

| Example No. | Ar | n | R | X | m.p. (°C) | NMR ($CDCl_3$, TMS) δ: |
|---|---|---|---|---|---|---|
| 2 | 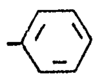 | 1 | 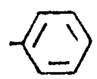 | 2-$CONH_2$ | 176-178 | |
| 3 | 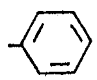 | 1 | 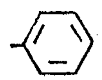 | 4-$CONH_2$ | 214-215 | |
| 4 | 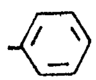 | 1 | 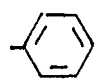 | 3-$SO_2NH_2$ | 67-69 | |
| 5 | 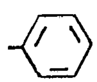 | 1 | 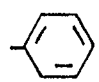 | 4-$SO_2NH_2$ | 189-191 | |
| 6 | 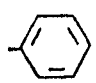 | 1 | 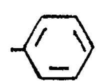 | 2-OH | | 8.68 (1H, s), 8.00 (1H, bs), 7.3-6.5 (14H,m), 3.90 (1H, t), 3.50 (1H, dd), 3.04 (1H, dd) |
| 7 | 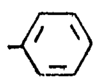 | 1 | 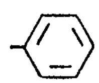 | 3-OH | 140-141 | |

(Continued)

| Example No. | Ar | n | R | X | m.p. (°C) | NMR ($CDCl_3$, TMS) δ: |
|---|---|---|---|---|---|---|
| 8 | 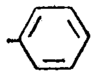 | 1 | 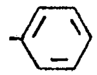 | 4-OH | 101-104 | |
| 9 | 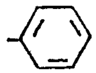 | 2 | 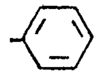 | 2-$CONH_2$ | | 11.30 (1H, bs), 8.53 (1H, dd), 7.5-7.0 (12H, m), 6.78 (1H, t), 6.34 2H, bs), 3.40 (1H, t), 2.7-1.9 (4H, m) |
| 10 | 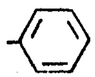 | 2 | 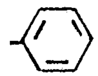 | 3-$CONH_2$ | 137-140 | |
| 11 | 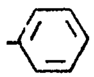 | 2 | 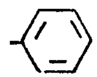 | 4-$CONH_2$ | | 10.04 (1H, bs), 7.9-7.6 (4H, m), 7.5-7.0 (12H, m), 3.68 (1H, t), 2.60 (2H, t), 2.5-1.9 (2H, m) |
| 12 | 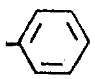 | 2 | 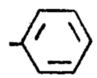 | 3-$SO_2NH_2$ | | 8.22 (1H, bs), 7.79 (1H, bs), 7.40 (1H, d), 7.3-6.8 (12H, m), 5.31 (2H, bs), 3.47 (1H, t), 2.6-1.9 (4H, m) |

(Continued)

| Example No. | Ar | n | R | X | m.p. (°C) | NMR ($CDCl_3$, TMS) δ: |
|---|---|---|---|---|---|---|
| 13 | 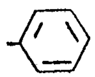 | 2 | 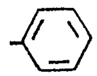 | 4-$SO_2NH_2$ | | 8.47 (1H, bs), 7.6-6.9 14H, m), 5.45 (2H, bs), 3.53 (1H, m), 2.6-1.9 (4H, m) |
| 14 | 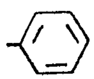 | 2 | 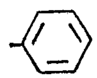 | 2-OH | | 8.95 (1H, bs), 8.31 (1H, s), 7.4-6.6 (14H, m), 3.57 (1H, t), 2.6-1.9 (4H, m) |
| 15 | 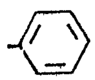 | 2 | 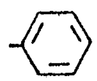 | 3-OH | 125-128 | |
| 16 | 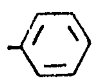 | 2 | 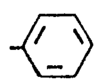 | 4-OH | 143-145 | |
| 17 | 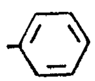 | 3 | 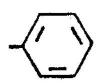 | 2-$CONH_2$ | | 8.57 (1H, d), 5.7-6.2 (2H, m), 3.50 (2H, t), 2.60 (2H, t) |
| 18 | 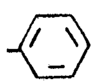 | 3 | 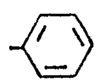 | 3-$CONH_2$ | 125-126 | |
| 19 | 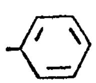 | 3 | 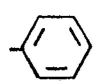 | 4-$CONH_2$ | 195-198 | |

(Continued)

| Example No. | Ar | n | R | X | m.p. (°C) | NMR ($CDCl_3$, TMS) δ: |
|---|---|---|---|---|---|---|
| 20 | 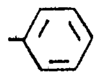 | 3 | 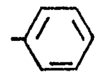 | 3-$SO_2NH_2$ | 62-64 | |
| 21 | 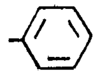 | 3 | 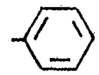 | 4-$SO_2NH_2$ | 123-126 | |
| 22 | 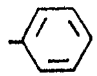 | 3 | 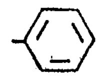 | 2-OH | | 8.79 (1H, bs), 8.00 (1H, bs), 7.3-6.5 (14H, m), 3.51 (1H, bt), 2.52 (2H, bt), 2.3-1.3 (4H, m) |
| 23 | 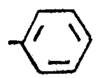 | 3 | 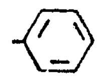 | 3-OH | 145-146 | |
| 24 | 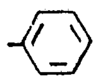 | 3 | 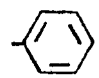 | 4-OH | 98-101 | |
| 25 | 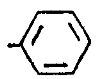 | 4 | 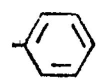 | 2-$CONH_2$ | | 8.56 (1H, d), 7.45-6.80 (14H, m), 6.15-5.65 (2H, br), 3.48 (1H, t), 2.57 (2H,t), 2.35-1.10 (8H, m) |

(Continued)

| Example No. | Ar | n | R | X | m.p. (°C) | NMR ($CDCl_3$, TMS) δ: |
|---|---|---|---|---|---|---|
| 26 | 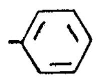 | 4 | 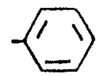 | 3-$CONH_2$ | 98-99 | |
| 27 | 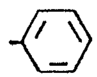 | 4 | 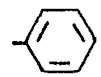 | 4-$CONH_2$ | 163.5-165.5 | |
| 28 | 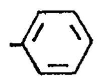 | 4 | 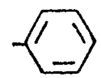 | 3-$SO_2NH_2$ | | 7.9-7.0 (14H, m), 5.10 (2H, s), 3.50 (1H, t), 2.57 (2H, t), 2.4-1.1 (6H, m) |
| 29 | 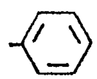 | 4 | 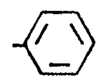 | 4-$SO_2NH_2$ | 125-127 | |
| 30 | 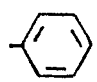 | 4 | 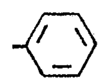 | 2-OH | | 8.73 (1H, bs), 7.74 (1H, bs), 7.4-6.7 (14H, m), 3.53 (1H, bt), 2.53 (2H, bt), 2.4-1.1 (6H, m) |
| 31 | 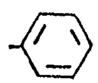 | 4 | 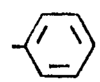 | 3-OH | 136-138 | |
| 32 | 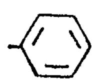 | 4 | 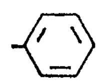 | 4-OH | 91-95 | |

(Continued)

| Example No. | Ar | n | R | X | m.p. (°C) | NMR ($CDCl_3$, TMS) δ: |
|---|---|---|---|---|---|---|
| 33 | 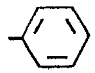 | 3 | 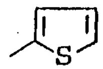 | 3-$CONH_2$ | 112-113 | |
| 34 | 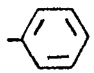 | 3 | 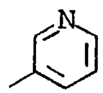 | 3-$CONH_2$ | 68-70 | |
| 35 | 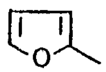 | 3 | 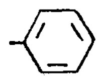 | 3-$CONH_2$ | 129-130 | |
| 36 | 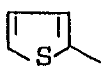 | 3 | 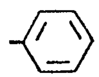 | 3-$CONH_2$ | 154.5-156 | |
| 37 | 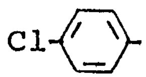 | 2 | 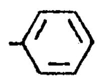 | 3-$CONH_2$ | 56-58 | |
| 38 | 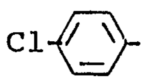 | 2 | 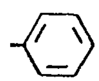 | 4-$SO_2NH_2$ | 178-179 | |
| 39 | 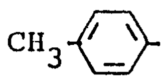 | 2 | 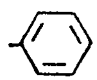 | 3-$CONH_2$ | 55-58 | |

(Continued)

| Example No. | Ar | n | R | X | m.p. (°C) | NMR ($CDCl_3$, TMS) δ: |
|---|---|---|---|---|---|---|
| 40 | $CH_3$-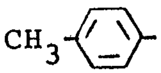 | 2 | 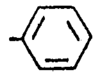 | 4-$SO_2NH_2$ | 140-150 | |
| 41 | 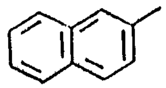 | 3 | 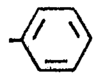 | 3-$CONH_2$ | | 8.1-6.2 (19H, m), 2.60 (2H, t), 2.5-1.4 (4H, m) |
| 42 | 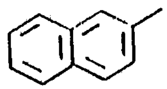 | 3 | 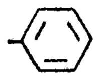 | 4-$SO_2NH_2$ | 68-71 | |

Example 43

To a mixture of alpha-(3-phenylpropyl)-N-(3-carboxyphenyl)benzeneacetamide (0.5 g), triethylamine (142 mg) and tetrahydrofuran (10 ml), ethyl chloroacetate (152 mg) was added at -10°C. After stirring for 30 minutes, a solution of diethylamine (196 mg) in tetrahydrofuran (10 ml) was added thereto at 0°C. After 1 hour, the reaction mixture was poured into water and extracted with ether. The organic layer was washed successively with dilute hydrochloric acid, an aqueous sodium bicarbonate solution and brine, dried over magnesium sulfate, concentrated and purified by chromatography on silica gel to give alpha-(3-phenylpropyl)-N-[3-(N,N-diethylcarbamoyl)phenyl]benzeneacetamide.

NMR ($CDCl_3$, TMS) $\delta$: 0.8 - 1.4 (6H, br), 1.4 - 2.5 (5H, m), 2.60 (2H, t), 2.9 - 3.8 (5H, m), 6.6 - 8.6 (13H, m), 8.41 (1H, s).

Example 44

To a mixture of alpha-(3-phenylpropyl)benzeneacetic acid (1.5 g), 2-aminobenzenesulfonamide (1 g) and hexamethylphosphortriamide (30 ml), thionyl chloride (0.84 ml) was added under ice-cooling. After 1 hour, the reaction mixture was poured into water and extracted with benzene. The organic layer was washed successively with dilute hydrochloric acid, an aqueous sodium carbonate solution and water, dried over magnesium sulfate, concentrated and purified by chromatography on silica gel to give alpha-(3-phenylpropyl)-N-(2-sulfamoylphenyl)benzeneacetamide.

NMR ($CDCl_3$, TMS) δ: 8.92 (1H, s), 8.13 (1H, d), 7.68 (1H, dd), 7.5 - 6.9 (12H, m), 4.56 (2H, s), 3.57 (1H, t), 2.63 (2H, t), 4.2 - 1.4 (4H, m).

Example 45

In the same manner as in Example 44 but using alpha-benzylphenylacetic acid and 2-aminobenzenesulfonamide as the starting materials, there was obtained alpha-benzyl-N-(2-sulfamoylphenyl)benzeneacetamide.

NMR ($CDCl_3$, TMS) δ: 8.84 (1H, s), 8.09 (1H, d), 7.74 (1H, dd), 7.5 - 6.9 (12H, m), 4.08 (2H, s), 4.0 - 3.4 (2H, m), 3.07 (1H, dd).

Example 46

A mixture of alpha-(3-phenylpropyl)-2-pyridineacetic acid ethyl ester (2.5 g) and 3-aminobenzamide (1.2 g) was heated at 200°C for 8 hours. After being allowed to cool, the reaction mixture was poured into water and extracted with ethyl acetate. The organic layer was washed with water, dried over magnesium sulfate, concentrated and purified by chromatography on silica gel to give alpha-(3-phenylpropyl)-N-(3-carbamoylphenyl)-2-pyridineacetamide (1.08 g). m.p., 142 - 143°C.

Example 47

In the same manner as in Example 1 but using (+)-alpha-(3-phenylpropyl)benzeneacetic acid ($[\alpha]_D^{20}$ = +54.3° ($CHCl_3$)) and 3-aminobenzamide as the starting materials, there was obtained (+)-alpha-(3-phenylpropyl)-N-(3-carbamoylphenyl)benzeneacetamide. m.p., 104 - 106°C. $[\alpha]_D^{20}$ = +71.7° ($CHCl_3$).

Example 48

In the same manner as in Example 1 but using (-)-alpha-(3-phenylpropyl)benzeneacetic acid ($[\alpha]_D^{20}$ = -55.6° ($CHCl_3$)) and 3-aminobenzamide as the starting materials, there was obtained (-)-alpha-(3-phenylpropyl)-N-(3-carbamoylphenyl)benzeneacetamide. m.p., 106 - 107°C. $[\alpha]_D^{20}$ = -62.9° ($CHCl_3$).

Example 49

In the same manner as in Example 1 but using (+)-alpha-(3-phenylpropyl)benzeneacetic acid ($[\alpha]_D^{20}$ = +54.3° ($CHCl_3$)) and 3-aminophenol as the starting materials, there was obtained (+)-alpha-(3-phenylpropyl)-N-(3-hydroxyphenyl)-benzeneacetamide. m.p., 114 - 115°C. $[\alpha]_D^{20}$ = +80.2° ($CHCl_3$).

Example 50

In the same manner as in Example 1 but using (-)-alpha-(3-phenylpropyl)benzeneacetic acid ($[\alpha]_D^{20}$ = -55.6° ($CHCl_3$)) and 3-aminophenol as the starting materials, there was obtained (-)-alpha-(3-phenylpropyl)-N-(3-hydroxyphenyl)-benzeneacetamide. m.p., 114 - 115°C. $[\alpha]_D^{20}$ = -72.3° ($CHCl_3$).

As stated above, the anilides (I) according to the invention show an excellent antihypoxic activity, which is supported, for instance, by the test example as set forth below.

Test Example

Antihypoxic activity:-

Test was carried out using the modification of the method described in Life Sciences, 13, 467 (1973).

Six ddy-mice weighing 20 - 27 g were used. Test compound was dissolved in saline or suspended in 0.5 % carboxymethylcellulose and injected intraperitoneally usually 30 minutes before the test at a dosage of 0.1 ml per 10 g of the bodyweight. Two mice were placed in a desiccator, the inside pressure of which was lowered to 190 mmHg within 2 to 3 seconds, and the survival time as well as the time interval between the start of the evacuation and the cessation of respiration was determined. The survival time of the mice which lived longer than 300 seconds was taken as 300 sec.

The compounds were effective when the means of the survival time was above 140 sec and the difference in the logarithms of the survival time between the treated group and the control group was significant ($p < 0.05$ by Students' t test).

The results are shown in Table 2 wherein the evaluation was made on the following standard:

-: ineffective at 30 mg/kg

+: effective at 30 mg/kg

++: effective at 10 mg/kg

Table 2

| Test compound | Remark | Antihypoxic activity |
|---|---|---|
| 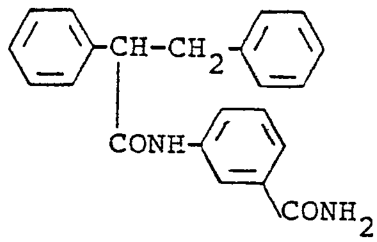 | Example 1 | ++ |
| 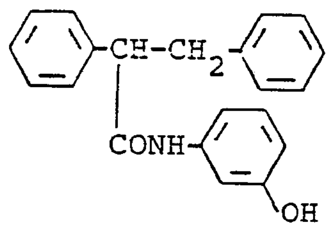 | Example 7 | ++ |
| 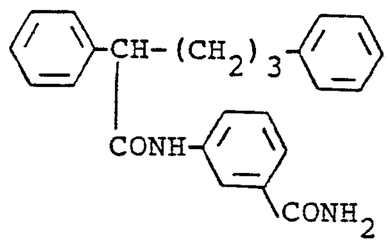 | Example 18 | ++ |
| 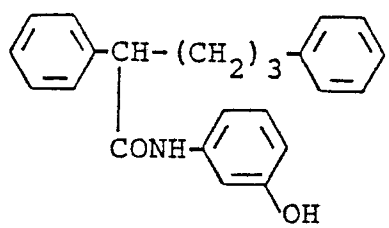 | Example 23 | ++ |
| 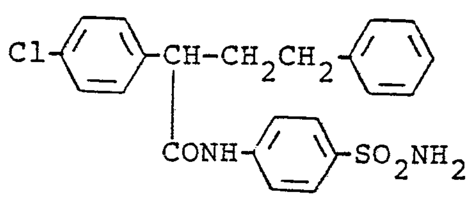 | Example 38 | ++ |

(Contiunued)

| Test compound | Remark | Antihypoxic activity |
|---|---|---|
| 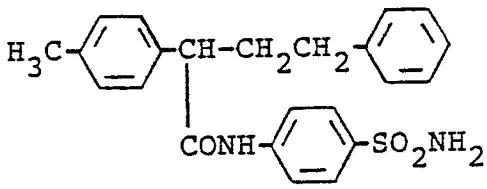 | Example 40 | ++ |
| Bencyclane | Commercial product | + |
| Papaverine | " | + |
| Vincamine | " | + |
| 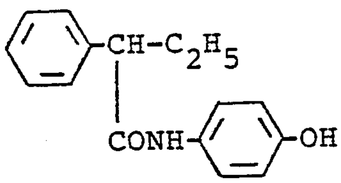 | Known product (see page 2) | - |

What is claimed is:

1. A compound of the formula I:

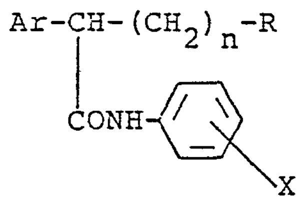

(I)

wherein Ar is a heterocyclic group or an aromatic hydrocarbon group optionally having one or two substituents selected from the group consisting of halogen, $C_1$-$C_4$ alkyl, $C_1$-$C_4$ alkoxy, nitro, cyano, di($C_1$-$C_4$)alkylamino, benzyloxy, hydroxy, $C_1$-$C_4$ alkylthio, $C_1$-$C_4$ alkylsulfinyl, $C_1$-$C_4$ alkylsulfonyl and $C_1$-$C_4$ alkanoylamino, X is a hydroxyl group or either one of the following groups: 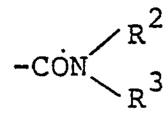 and 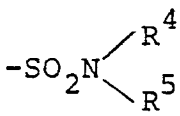 (in which $R^2$, $R^3$, $R^4$ and $R^5$ are each independently hydrogen or $C_1$-$C_4$ alkyl or, when $R^2$ and $R^3$ or $R^4$ and $R^5$ are taken together with the adjacent nitrogen atom to which they are attached, they represent pyrrolidino, piperidino, morpholino, N-($C_1$-$C_4$)alkylpiperazino or N-phenylpiperazino), R is an aromatic hydrocarbon or heterocyclic group and n is an integer of 1 to 4, and its acid addition salt.

2. The compound according to claim 1, wherein Ar is a phenyl group optionally having halogen or $C_1$-$C_4$ alkyl, a naphthyl group, a pyridyl group, a furyl group or a thienyl group, X is a hydroxyl group or either one of the following groups: 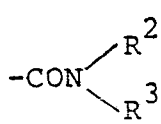 and 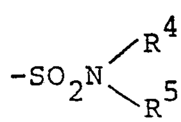 (in which $R^2$,

$R^3$, $R^4$ and $R^5$ are each hydrogen or $C_1$-$C_4$ alkyl) and R is a phenyl, pyridyl or thienyl group.

3. The compound according to claim 1, wherein Ar is a phenyl group optionally having halogen or $C_1$-$C_4$ alkyl, X is a hydroxyl group or either one of the following groups: $-CONH_2$ and $-SO_2NH_2$, R is a phenyl group and n is an integer of 1 to 3.

4. The compound according to claim 1, wherein Ar is a phenyl group, X is $-CONH_2$, R is a phenyl group and n is an integer of 1.

5. The compound according to claim 1, wherein Ar is a phenyl group, X is $-CONH_2$, R is a phenyl group and n is an integer of 3.

6. The compound according to claim 1, wherein Ar is a phenyl group, X is a hydroxyl group, R is a phenyl group and n is an integer of 1.

7. The compound according to claim 1, wherein Ar is a chlorophenyl group, X is $-SO_2NH_2$, R is a phenyl group and n is an integer of 2.

8. The compound according to claim 1, wherein Ar is a methylphenyl group, X is $-SO_2NH_2$, R is a phenyl group and n is an integer of 2.

9. A process for producing a compound of the formula I:

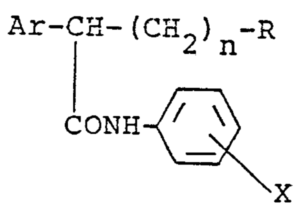

(I)

wherein Ar is a heterocyclic group or an aromatic hydrocarbon group optionally having one or two substituents selected from the group consisting of halogen, $C_1$-$C_4$ alkyl, $C_1$-$C_4$ alkoxy, nitro, cyano, di($C_1$-$C_4$)alkylamino, benzyloxy, hydroxy, $C_1$-$C_4$ alkylthio, $C_1$-$C_4$ alkylsulfinyl, $C_1$-$C_4$ alkylsulfonyl and $C_1$-$C_4$ alkanoylamino, X is a hydroxyl group or either one of the following groups: 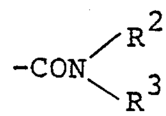 and 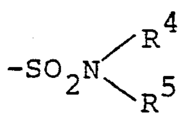 (in which $R^2$, $R^3$, $R^4$ and $R^5$ are each independently hydrogen or $C_1$-$C_4$ alkyl or, when $R^2$ and $R^3$ or $R^4$ and $R^5$ are taken together with the adjacent nitrogen atom to which they are attached, they represent pyrrolidino, piperidino, morpholino, N-($C_1$-$C_4$)alkylpiperazino or N-phenylpiperazino), R is an aromatic hydrocarbon or heterocyclic group and n is an integer of 1 to 4, and its acid addition salt, which comprises reacting a compound of the formula II:

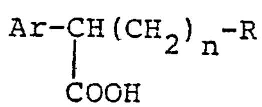

(II)

wherein Ar, R and n are each as defined above, or its reactive derivative at -COOH, with a compound of the formula (III):

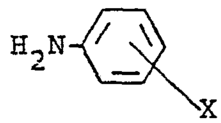

(III)

wherein X is as defined above.

10. A process for producing a compound of the formula I:

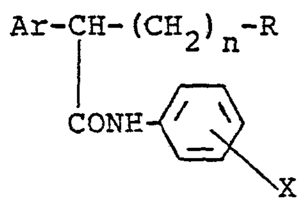

(I)

wherein Ar is a heterocyclic group or an aromatic hydrocarbon group optionally having one or two substituents selected from the group consisting of halogen, $C_1$-$C_4$ alkyl, $C_1$-$C_4$ alkoxy, nitro, cyano, di($C_1$-$C_4$)alkylamino, benzyloxy, hydroxy, $C_1$-$C_4$ alkylthio, $C_1$-$C_4$ alkylsulfinyl, $C_1$-$C_4$ alkylsulfonyl and $C_1$-$C_4$ alkanoylamino, X is a hydroxyl group or either one of the following groups: 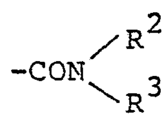 and 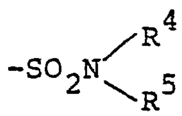 (in which $R^2$, $R^3$, $R^4$ and $R^5$ are each independently hydrogen or $C_1$-$C_4$ alkyl or, when $R^2$ and $R^3$ or $R^4$ and $R^5$ are taken together with the adjacent nitrogen atom to which they are attached, they represent pyrrolidino, piperidino, morpholino, N-($C_1$-$C_4$)alkylpiperazino or N-phenylpiperazino), R is an aromatic hydrocarbon or heterocyclic group and n is an integer of 1 to 4, and its acid addition salt, which comprises reacting the reactive derivative of either one of the compounds of the formulas:

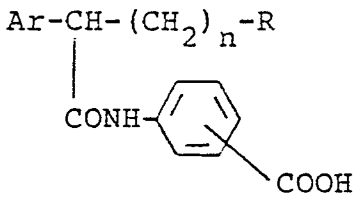

(IV)

and

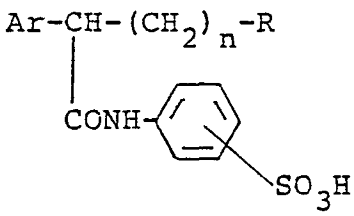

(V)

wherein Ar, R and n are each as defined above with an amine of either one of the formulas:

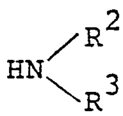 (VI) or 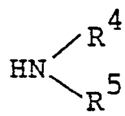 (VII)

wherein $R^2$, $R^3$, $R^4$ and $R^5$ are each as defined above.

11. A pharmaceutical composition which comprises as an active ingredient the compound according to any of claims 1 to 8 and a pharmaceutically acceptable carrier or diluent.

12. Use of the compound as claimed in any of claims 1 to 8 for preparing a cerebral metabolism stimulant or a nootropic agent.